# EUROPEAN PATENT SPECIFICATION

(11) **EP 4 306 090 B1**
(45) Date of publication and mention of the grant of the patent: **10.09.2025**
(21) Application number: 22185328.6
(22) Date of filing: 15.07.2022
(51) Int. Cl.: A61F 13/15, B01D 46/26, B01D 46/682

(54) **PROCESS LINE FOR MANUFACTURING ABSORBENT ARTICLES**
PROZESSLINIE ZUR HERSTELLUNG VON SAUGFÄHIGEN ARTIKELN
LIGNE DE TRAITEMENT POUR LA FABRICATION D'ARTICLES ABSORBANTS

(43) Date of publication of application: 17.01.2024
(73) Proprietor: Ontex BV, 9255 Buggenhout (BE); Ontex Group NV, 9320 Erembodegem (BE)
(72) Inventor: HEEGE, Thomas, 56761 Düngenheim (DE)
(74) Representative: Saurat, Thibault

(56) References cited:
- US-A1- 2014 110 052

## Description

### TECHNICAL FIELD

The present disclosure relates to a process line for manufacturing absorbent articles.

### BACKGROUND

A process line for manufacturing absorbent articles, namely disposable hygiene products such as diapers, sanitary napkins or incontinence pants comprises several stations to carry out different steps. Some of these steps require vacuum to ensure the suction or the blowing of components of the absorbent articles, e.g. maintaining absorbent core onto a drum or depositing a cut-out onto a web. Providing vacuum at different stations implies using a sophisticated air circulation system that can be costly in terms of energy with eventually an air filtering system.

The disclosure thereto aims to provide an apparatus which ensures an efficient and optionally reliable vacuum throughout the process line for manufacturing absorbent articles at an optimized energy consumption.

### SUMMARY OF THE INVENTION

The present disclosure provides a process line for manufacturing absorbent articles comprising a plurality of assembly modules, the process line comprising at least one channel fluidically connecting at least one assembly module and a main pump, said main pump being arranged downstream, with respect to the airflow, of said assembly module wherein at least one booster pump is arranged between an assembly module and said main pump, said at least one channel fluidically connecting said assembly module, booster pump and main pump, the volume flow rate of the main pump is at least twenty times greater than any of the booster pumps.

According to an embodiment, the number of assembly modules is greater than the number of booster pumps.

The maximum volume flow rate of the main pump is at least twenty times greater than the maximum volume flow rate of any of the booster pumps. Similarly, the functioning volume flow rate of the main pump is at least twenty times greater than the functioning volume flow rate of any of the booster pumps, functioning volume flow rate meaning while in function or while the process line is running and manufacturing absorbent articles.

According to an embodiment, the process line comprises a filtering apparatus arranged upstream of main pump, with respect to the airflow.

According to an embodiment, the volume flow rate of the main pump is comprised between 20 000 m³/h and 50 000 m³/h and the volume flow rate of any booster pump is comprised between 100 m³/h and 1 000 m³/h.

According to an embodiment, the process line comprises at least two channels.

According to an embodiment, said at least two channels comprise an interconnection where two channels fuse into a single channel, the interconnection being arranged upstream of the filtering apparatus.

According to an embodiment, the process line comprises a basic pump arranged upstream of the main pump and downstream of the booster pump, said at least one channel fluidically connecting said assembly module, booster pump, basic pump and main pump.

According to an embodiment, the volume flow rate of the main pump is between 0.8 and 2 times greater than the volume flow rate of the basic pump, the volume flow rate of the basic pump being comprised between 20 000 m³/h and 40 000 m³/h. More precisely, the maximum volume flow rate of the main pump is between 0.8 and 2 times greater than the maximum volume flow rate of the basic pump. Similarly, the functioning volume flow rate of the main pump is between 0.8 and 2 times greater than the functioning volume flow rate of the basic pump.

According to an embodiment, the process line comprises communication means between the booster pumps and the main pump and/or basic pump wherein a booster pump is configured to send electronic signals to the main pump and/or basic pump.

According to an embodiment, the filtering apparatus comprises:
a housing comprising at least one inlet;
a filtering media arranged downstream with respect to the airflow of the at least one inlet ;
a support structure comprising a cylindrical drum comprising a first and second longitudinal end, the filtering media being secured to said cylindrical drum;
a motor device to rotate the cylindrical drum; and
a first pump;
wherein the motor device comprises a driving mean and the cylindrical drum comprises the corresponding driven mean arranged at the periphery of one of the longitudinal ends of the cylindrical drum, the driving mean actuating the driven mean and cylindrical drum.

According to an embodiment, the driving mean comprises a geared plate and the driven mean comprises a ring gear, the teeth of the geared plate engaging the teeth of the ring gear.

According to an embodiment, the filtering apparatus comprises a recycling pump arranged to suck up a lesser portion of the air incoming in the housing than the main pump.

According to an embodiment, the filtering apparatus comprises a least one nozzle arranged in proximity of the filtering media and at least one duct linking said nozzle to the second pump, wherein two or more nozzles are not aligned with respect to the longitudinal axis of the cylindrical drum.

All of these embodiments mentioned above can be taken individually or in combination.

Further embodiments are described below and in the Document US 2014/110052 represents relevant prior art in the technical field.

### DESCRIPTION OF FIGURES

The drawings and figures are illustrative in nature and not intended to limit the subject matter defined by the claims. The following detailed description can be understood when read in conjunction with the following drawings, where like structure is indicated with like reference numerals in which:
**FIG. 1** illustrates a schematic representation of an embodiment of the process line with a diagram flow chart illustrating the airflow;
**FIG. 2** illustrates a view in perspective of the filtering apparatus according to the disclosure;
**FIG. 3** illustrates a view of a portion of the filtering apparatus, namely the driving mechanism;
**FIG. 4** illustrates a schematic representation of another embodiment of the process line with a diagram flow chart illustrating the airflow.

### DETAILED DESCRIPTION OF THE INVENTION

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, tampons, diaper holders and liners, pantiliners, sanitary napkins and the like, as well as surgical bandages and sponges. Disposable absorbent articles comprise a liquid pervious topsheet, a liquid impervious backsheet joined to the topsheet, and an absorbent core positioned and held between the topsheet and the backsheet. The absorbent article, or absorbent assembly, may also include other components, such as liquid wicking layers, liquid intake layers, liquid distribution layers, transfer layers, barrier layers, wrapping layers and the like, as well as combinations thereof.

"Absorbent material" as used herein refers to the material constituting the absorbent core of the absorbent article. Examples of commonly occurring absorbent materials are cellulosic fluff pulp, tissue layers, highly absorbent polymers or superabsorbent polymer particles (SAP), absorbent foam materials, absorbent nonwoven materials or the like. It is common to combine cellulosic fluff pulp with superabsorbent polymers in an absorbent material.

"Comprise," "comprising," and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The terms "downstream" and "upstream" as used herein are with respect to the airflow stream, meaning that the airflow goes from an upstream position to a downstream position.

The terms "air filtering apparatus" and "filtering apparatus" refer to the same apparatus, similarly as "inlet" and "air inlet" reefing to the same inlet.

The terms "height", "bottom", "ceiling", "ground" are all in reference to the apparatus and its components once installed and in a functioning state.

The term "nonwoven fabric" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

The term "volume flow rate", or "volumetric flow rate" or also referenced as "air capacity" in reference to a pump such as a centrifugal fan, means herein the volume of fluid, meaning air eventually loaded with absorbent material, which can pass through said pump per unit time. It is meant herein as the functioning volume of air passing through said pump while in a functioning mode, meaning when the process line is running. Alternatively, the term "volume flow rate" can also be understood as the maximum volume of air that can pass through said pump in a given time e.g. 1 hour.

The recitation of numerical ranges by endpoints includes all numbers and fractions subsumed within that range, as well as the recited endpoints.

In the following detailed description of the preferred embodiments, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

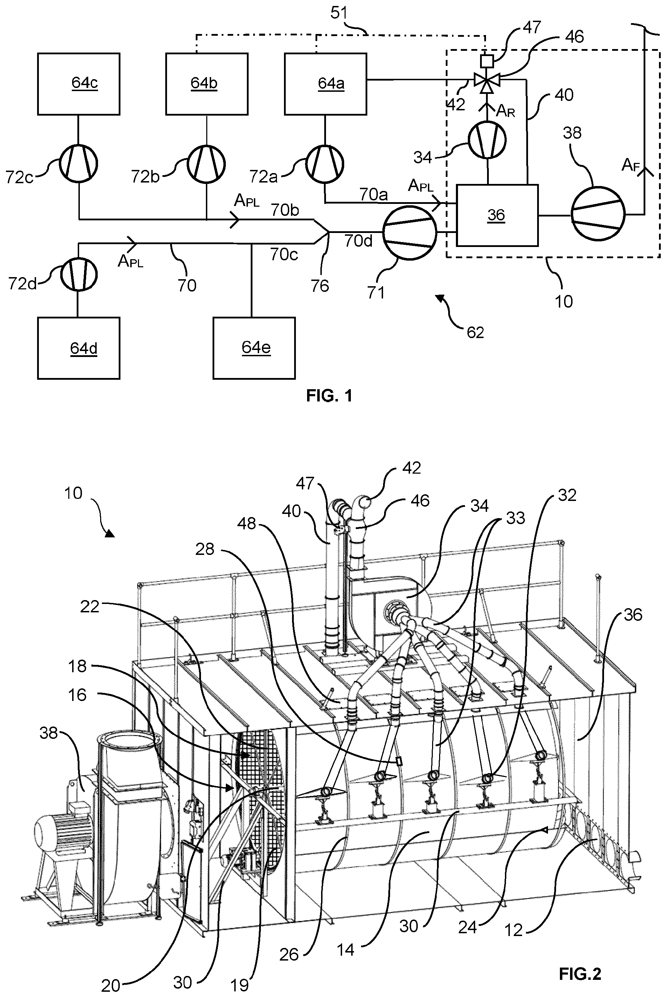

FIG. 1 is a diagram illustration of a process line 62 for manufacturing absorbent article and the air circulation system in said process line according to one embodiment. The process line 62 comprises a plurality of assembly modules 64 here five assembly module 64 as illustrated in FIG. 1. As explained previously, absorbent articles comprise a liquid pervious topsheet, a liquid impervious backsheet joined to the topsheet, and an absorbent core positioned and held between the topsheet and the backsheet. The absorbent article, or absorbent assembly, may also include other components, such as liquid wicking layers, liquid intake layers, liquid distribution layers, transfer layers, barrier layers, wrapping layers and the like, as well as combinations thereof. These different components are assembled altogether at different assembly modules 64. The assembly module can be simple apparatus with a limited number of elements such as a roller applying a web, *e.g.* the topsheet, onto another element, *e.g.* the absorbent core, or they can be more complex apparatus comprising a greater number of elements such as the drum former where the absorbent core is assembled, *e*.*g*. by stacking absorbent material between two core wraps, or an insert repitch unit. Some of these assembly modules 64 require a vacuum source to either suction and maintain a component on a surface or blow and expel a component from on a surface.

As such the process line 62 comprises an air circulation system to manage the circulation of airflows. The process line 62 comprises at least one channel 70, or duct, fluidically connecting at least one assembly module 64 and a main pump 38. The main pump 38 is a mean to move fluid, namely air eventually charged with absorbent material, it can be for example a centrifugal fan. In other words, an assembly module 64 and the main pump 38 are in fluidic connection, specifically in air connection. The main pump 38 is arranged downstream with respect to the airflow direction, of the plurality of assembly modules 64. The energy consumption of the main pump 38 varies depending on the number of assembly modules 64. To this effect, the process line 62 comprises at least one booster pump 72 arranged between an assembly module 64 and said main pump 38. The at least one channel 70 fluidically connecting said assembly module 64, eventual booster pump 72 and a main pump 38. The booster pump(s) 72 is also a mean to move fluid, namely air eventually charged with absorbent material, it can be for example also a centrifugal fan.

As illustrated in FIG. 1, the process line 62 comprises here three channels 70 : a first channel 70a that is fluidically connecting a first assembly module 64a a first booster pump 72a and the main pump 38, a second channel 70b that is fluidically connecting a second and third assembly modules 64b,64c, a respective second and third booster pump 72b,72c and the main pump 38 and a third channel 70c that is fluidically connecting a fourth and fifth assembly module 64d,64e a fourth booster pump 72d and the main pump 38. The process line 62 can also be seen as comprising one channel 70 with three sub-channels 70a, 70b, 70c that are connected in the housing 36 (that will be described hereunder), or even as comprising more than ten channels, for instance the air circulation portion between the assembly module 64c and the booster pump 72c can be considered as a channel. A channel 70 is to be understood herein as a duct or pipe, connecting two or more components of the process line 62 such as assembly modules 64 ; pumps 38,71,72 ; housing 36 ; filtering apparatus 10 ; interconnections 76, *etc.*

For embodiments of the process line 62 comprising more than one channel 70, these channels 70 can be fluidically connected. In other words, the channels 70 can comprise an interconnection 76, or intersection or a bridging portion, where the two or more channels 70 connect, or fused together, and form only one channel. For instance, as illustrated in FIG. 1, the second and third channels 70b,70c comprise an interconnection 76 and fuse into one channel 70d.

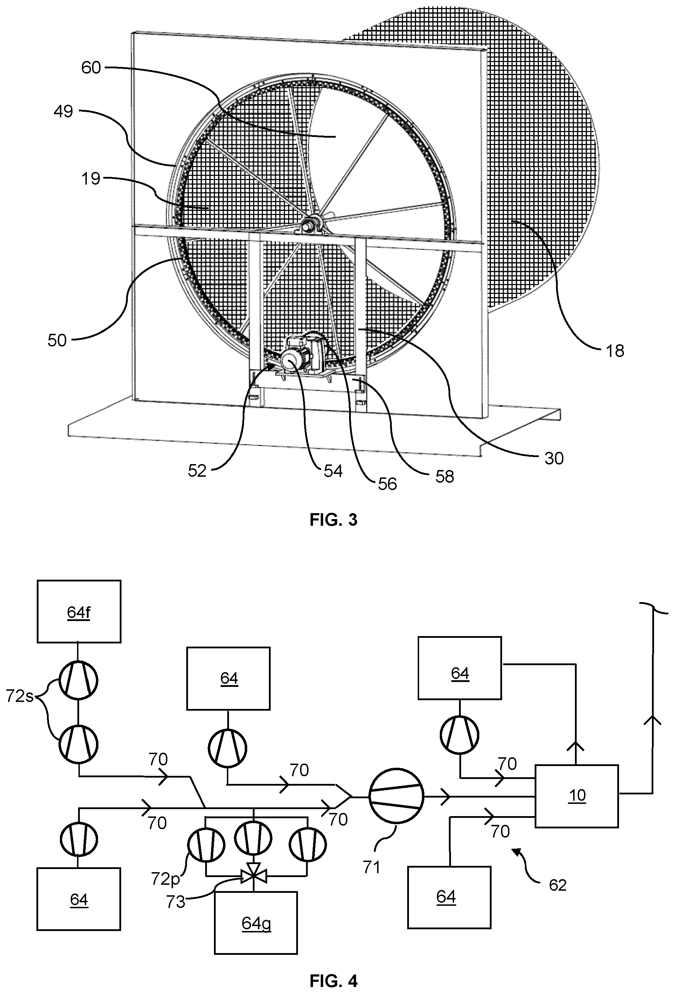

The process line 62 also comprises an air filtering apparatus 10. The filtering apparatus 10, or air filtering installation, for filtering the air coming from the process line 62 configured to manufacture absorbent articles such as diapers, incontinence or training pants, sanitary napkins, underpads for example, and for recycling the absorbent material present in said air. As illustrated in FIG. 2 and FIG. 3, the filtering apparatus 10 for filtering air coming from an absorbent articles manufacturing process line 62, said filtering apparatus 10 comprises:
- a housing 36 comprising at least one inlet 12;
- a filtering media 14 arranged downstream with respect to the airflow of the at least one inlet 12;
- a support structure 16 comprising a cylindrical drum 18 comprising a first and second longitudinal end 49,60, the filtering media 14 being secured to said cylindrical drum 18;
- a motor device 52 to rotate the cylindrical drum 18; and
- a first pump 38.

According to the disclosure, the motor device 52 comprises a driving mean 56 and the cylindrical drum 18 comprises the corresponding driven mean 50 arranged at the periphery of one of the longitudinal ends 49,60 of the cylindrical drum 18, the driving mean 56 actuating the driven mean and cylindrical drum 18. In other words, the motor device 52 comprises an actuator 56 comprising at least one male or female rotation member, *i.e.* the driving mean(s), and the cylindrical drum 18 comprises the corresponding, or complementary, female or male rotation member, *i.e.* the driven mean(s), for driving engagement therewith, the male or female rotation member(s) of the cylindrical drum 18 being arranged at the periphery of one of the longitudinal ends 49,60 of the cylindrical drum 18 and the male or female rotation member of the actuator 56 actuating the male or female rotation member of the cylindrical drum 18.

The air filtering apparatus 10 preferably comprises a plurality of inlets 12 or air inlets, for example five air inlets 12 as illustrated in FIG. 2, or it can be two air inlets as illustrated in FIG. 1. The disclosure is not limited to this number of inlets 12. The filtering apparatus 10 may comprise two, three, four, six, seven, eight, nine, ten or more inlets 12.

As illustrated in FIG. 1, the housing 36 comprising the air filtering media 14, or the housing 36 in which the filtering media 14 is arranged, is arranged between the main pump 38 and the assembly module(s) 64 and/or booster pump(s) 72. In other words, the main pump 38 is preferably arranged downstream, with respect to the airflow, of the assembly module(s) 64 and booster pump(s) 72 and housing 36. The main pump 38 could be arranged upstream of the housing according to an embodiment.

The filtering apparatus 10 comprises a support structure 16 to carry, support or maintain, the filtering media 14 in a steady position, the filtering media 14. For example, the support structure 16 comprises a cylindrical drum 18 elongated, or extending, in a longitudinal direction and comprising a grid 19, latticework or mesh 19 or in other words a weblike pattern or construction 19 that can retain the filtering media 14 while being air-permeable. The mesh 19 defines the lateral surface of the cylindrical drum 18. The cylindrical drum 18 can further comprise a central shaft 20 around which the cylindrical drum 18 rotates and optionally several reinforcements 22 such as rods, or arms, linking the central shaft 20 to the cylindrical drum 18 or bridging two diametrically opposed points of the cylindrical drum 18. The reinforcements 22 can extend in the radial direction and/or in the longitudinal direction of the cylindrical drum 18. The filtering media 14 is fastened, or secured, onto the support structure 16. The filtering media 14 can for example comprise a zipper system 24 comprising a slider connecting two transversal ends of the filtering media 14 around the cylindrical drum 18, the transversal ends comprising two rows of teeth that are designed to interlock thereby joining the transversal ends together around the cylindrical drum 18. The filtering media 14 can also be fastened onto the cylindrical drum 18 with straps 26 and corresponding buckles 28. As illustrated in FIG. 2, the air filtering apparatus 10 comprises here five straps 26 to further secure the filtering media 14 to the cylindrical drum 18 in addition of the zipper 24.

The central shaft 20 when present can extend along the entire length of the cylindrical drum 18, or as illustrated in FIG. 2, the central shaft can extend along a portion of the longitudinal axis of the cylindrical drum 18.

The filtering apparatus 10 may also comprise a frame 30 to maintain the support structure 16. The frame 30, or space frame, comprises a plurality of struts and/or reinforcements such as rods or arms forming a truss-like structure to carry and support the support structure 16, in particular the cylindrical drum 18.

The air filtering apparatus 10 comprises at least one nozzle 32, for example, two, three, four, five, six, seven, eight, nine, ten or more nozzles 32, here five nozzles are illustrated in FIG.1. The one or more nozzles 32 are linked through ducts 33 to a pump 34, that will be referenced as the recycling pump 34 henceforth, enabling a suction of air and absorbent material contained in said air. The plurality of nozzles 32 are arranged in close proximity to the filtering media 14. More precisely, the recycling pump 34 is arranged and configured to suck up a portion, meaning not the entirety, of the air incoming in the housing 36.

Each nozzle 32 comprises an inlet which is preferably convergent with respect to the airflow direction where the cross section of the nozzle is narrowing down, or decreasing, from a greater cross section to a lesser cross section in the direction of the flow. By being convergent and in close proximity of the filtering media 14, the surface of suction of the nozzle 32 is greater thereby increasing the amount of absorbent material to be suctioned by the recycling pump 34. In other words, the greater cross section 32 of the nozzle is closest to the filtering media 14 whereas the lesser cross section of the nozzle 32 is closest to the duct 33 with respect to the airflow direction.

The air filtering apparatus 10 also comprises a housing 36 defining an inner volume with a set of walls, for example four walls and a ground wall and ceiling wall and optionally partition walls within said inner volume. The filtering media 14, support structure 16, nozzles 32 and frame 30 are arranged at least partially within said inner volume. The housing 36 enables to contain the air to be filtered within said inner volume and directs the airflow to pass through the filtering media 14. The process line 62 and more specifically the air filtering apparatus 10 comprises a pump 38, that will be referenced as the main pump 38 henceforth, enabling a suction of air and absorbent material contained within said air. The main pump 38 is preferably arranged downstream of the filtering media 14 with respect to the airflow. The main pump 38 comprises an inlet that is in fluidic connection with the inner volume defined by the housing 36 and an outlet that is arranged outside, or in the exterior of, the housing 36, or at least in fluidic connection with the exterior of the housing.

The main pump 38, the booster pumps 72 and the recycling pump 34 are all means for moving fluids, in this case air eventually charged, or loaded, with absorbent material. The main pump 38 can be referenced as the first pump 38 and the recycling pump 34 can be reference as the second pump 38. The present disclosure is not limited to the nature of these pumps. The main pump 38, booster pumps 72 and recycling pump 34 can be selected from and not limited to centrifugal pumps such as centrifugal fans, axial-flow pumps, diaphragm pumps, impeller pumps, screw pumps, booster pumps, canned motor pump, circulator pumps, single or multistage pumps and turbine pumps. The main pump 38 is a pump ensuring vacuum throughout the absorbent articles manufacturing process line 62 and has preferably a higher volume flow rate, or air capacity, then the recycling pump 34. In other words, the main pump 38 enables a greater flow than the recycling pump 34, *i.e.* the flow of air passing through the main pump 38 is greater than the flow passing through the recycling pump 34. The volume flow rate, or air capacity, of the main pump 38 is at least ten times greater than the volume flow rate of the recycling pump 34.

The volume flow rate, or air capacity, of the main pump 38 is at least twenty times, more preferably between 25 and 50 times greater than the volume flow rate of the recycling pump 34. For example, the recycling pump 34 has a volume flow rate of about 1 000 m³/h whereas the main pump 38 has a volume flow rate between about 30 000 m³/h and about 40 000 m³/h. The main pump 38 and the recycling pump 34 are preferably of the same nature, for example, the main pump 38 and the recycling pump 34 are both centrifugal fans. Alternatively the main pump 38 and the recycling pump 34 can be of different nature. For instance, given that the main pump 38 is destined to move air and the recycling pump 34 is destined to move air and absorbent material, the recycling pump can have specific seals and an impeller adapted to avoid clogging. Given that the main pump 38 has a greater air capacity than the recycling pump 34, the main pump 38 will suck in the majority of the air incoming in the housing 36 and the recycling pump 34, through the nozzle will suck a lesser portion of that air than the main pump 38. In other words, given the arrangement and the air capacities of the pumps 34,38, the majority of the air incoming in the housing 36 flows through the main pump 38 and a minority of that incoming air flows through the recycling pump 34.

Similarly, the main pump 38 has preferably a higher volume flow rate, or air capacity, then any of the booster pumps 72. In other words, the main pump 38 conveys a greater volume flow than any of the booster pumps 72, *i.e.* the flow of air passing through the main pump 38 is greater than the flow passing through any of the booster pumps 72. The volume flow rate, or air capacity, of the main pump 38 is at least twenty times greater than the volume flow rate, or air capacity of any of the booster pumps 72. Preferably, the volume flow rate, or air capacity, of the main pump 38 is at least thirty times, more preferably between 30 and 250 times greater than the air capacity of any of the booster pumps 72, more preferably between 35 and 200 times greater. For example, the booster pumps 72 have a volume flow rate ranging from 200 m³/h to 800 m³/h whereas the main pump 38 has a volume flow rate between about 30 000 m³/h and about 40 000 m³/h. The main pump 38 and each of the booster pumps 72 are preferably of the same nature, for example, the main pump 38 and each booster pump 72 are centrifugal fans. Alternatively, the main pump 38 and a booster pump 72 can be of different nature. For instance, given that the main pump 38 is destined to move air and a booster pump 72 is destined to move air charged with absorbent material, a booster pump 72 can have specific seals and an impeller adapted to avoid clogging. A fluid, *i.e.* air eventually loaded with absorbent material, sucked in and expelled by a pump will gain in velocity and in pressure. Given that the booster pumps 72 and the main pump 38 are fluidically connected, with respect of the airflow, the main pump 38 does not need to function at higher power to ensure a good vacuum throughout the process line 62 since the booster pump can ensure some of the vacuum.

According to an embodiment, in order to further ease the workload of the main pump 38, in other words to lower the energy consumption of the main pump 38, the process line 62 can further comprise a basic pump 71. The basic pump 71 is a mean to move fluid, namely air eventually loaded with absorbent material, it can be for example a centrifugal fan. The basic pump 71 can be selected from and not limited to centrifugal pumps such as centrifugal fans, axial-flow pumps, diaphragm pumps, impeller pumps, screw pumps, booster pumps, canned motor pump, circulator pumps, single or multistage pumps and turbine pumps. The basic pump 71 is arranged upstream of the main pump 38 with respect to the airflow direction or in other words, the basic pump 71 is arranged upstream of the filtering apparatus 10. As illustrated in FIG. 1, some assembly modules 64, 64b, 64c, 64d, 64e, the basic pump 71 and the main pump 38, or air filtering apparatus 10, are in fluidic connection, specifically in air connection. The main pump 38 is arranged downstream with respect to the airflow direction, of the plurality of assembly modules 64, with the basic pump 71 being arranged in between some of the assembly module 64, 64b, 64c, 64d, 64e with corresponding booster pump 72. The process line 62 comprises at least one booster pump 72 arranged between one assembly module and the basic pump 71. The at least one channel 70 fluidically connecting said assembly module 64, booster pump 72 basic pump 71 and a main pump 38.

The basic pump 71 and the main pump 38 are both pumps ensuring a strong vacuum in the process line 62 and thus can have a volume flow rate, or air capacity, in the same range. Preferably, the main pump 38 enables a greater flow than the basic pump 71, *i.e.* the flow of air passing through the basic pump 71 is greater than the flow passing through any of the booster pumps 72. The volume flow rate, or air capacity, of the basic pump 71 is at between 0.8 and 2 times greater than the volume flow rate, or air capacity of any of the basic pump 72. Preferably, the volume flow rate, or air capacity, of the main pump 38 is between 1.02 and 1.5 times greater than the volume flow rate of the basic pump 71. For example, the basic pump 71 has a volume flow rate of about 29 000 m³/h whereas the main pump 38 has a volume flow rate between about 30 000 m³/h and about 40 000 m³/h. The basic pump 71 and the main pump 38 are preferably of the same nature, for example, the basic pump 71 and the main pump 38 are both centrifugal fans.

Following the airflow, the air coming from the process line 62 and the assembly modules 64, containing absorbent material such as cellulosic fibers e.g. fluff and/or superabsorbent particles, enters the filtering apparatus 10, namely enters within the inner volume defined by the housing 36 through the one or more air inlets 12. The air then passes through the filtering media 14 and is then extracted from the inner volume by the main pump 38. The filtering media 14 filters the air so that the absorbent material (cellulosic fibers and/or SAP) contained in the air is retained within, or on the surface of, the filtering media 14.

The one or more nozzles 32 being placed in close proximity to the filtering media 14 enable to extract said absorbent material from the filtering media 14. The recycling pump 34 comprises one inlet that is linked one or more nozzles 32 and a first and second outlets 40,42. The first outlet 40 is in fluidic connection with the inner volume defined by the housing 36. In other words, the recycling pump 38 can re-inject the air and absorbent material within the air filtering apparatus 10 and ensure a re-filtering of the air. The second outlet 42 is in fluidic connection with the process line 62, specifically with one of the assembly module 64, namely with the absorbent core forming station of the process line 62 where the recycled absorbent material can be re-used and arranged within an absorbent article. The recycling pump 34 or outlet duct of said pump 34 preferably comprises a valve 46 to guide the air through one outlet or the other. When the process line for manufacturing absorbent articles is running, the valve 46 guides the air and absorbent material towards the second outlet 42 to recycle the absorbent material whereas if the process line is temporarily stopped, the valve 46 guides the air and absorbent material toward the first outlet 40 to refilter the air and ensure a closed air loop. The valve 46 is preferably a three-port valve such as a three-way ball valves come with a L-shaped fluid passageways inside the rotor. The valve 46 also comprises an actuator 47 to control the direction of the airflow, preferably the valve 46 comprises an electromechanical actuators such as an electric motor or solenoid to enable an automatic control or a remote control. For instance, when the process line 62 is temporarily stopped, more specifically when one or more assembly module 64 is temporarily stopped it can send, or emit, an electronic signal 51 to the actuator 47 to orientate the airflow towards the first outlet 40 and housing 36. The present disclosure is not limited to electronic signal 51 only, there can be alternative or complementary additional control feedbacks 51 such as an operator's input (manual or electronic input) and/or a motor speed feedback and/or the output signal emitted by the rotational speed sensor and/or the output signal emitted by an emergency shut-down button and/or the output signal emitted by an image detection device, or a quality control device.

The airflow and process line 62 and filtering apparatus 10 are schematically illustrated in FIG. 1. The air A_{PL} coming from the process line 62 in which absorbent articles are manufactured, namely the air A_{PL} coming from any of the assembly module 64 is conveyed towards filtering apparatus 10 and enters the inner volume of the housing via the inlet(s) 12, here via two inlets. The air A_{PL} entering the inlets 12 contains an average amount of absorbent material. The air is then filtered, air A_{F}, and is conveyed towards the exterior by the main pump 38. This filtered air A_{F} contains none or an infinitesimal amount of absorbent material. Alternatively, the recycled absorbent material is suctioned by the nozzles 32 and recycling pump 34 and the recycled air A_{R} is either sent back to the absorbent articles manufacturing process line 62 via the second outlet 42 or sent back to the filtering media 14 via the first outlet 41. The recycled absorbent material-laden air A_{R} contains an average or greater amount of absorbent material.

The housing 36 can comprise one or more transparent, or see-through, panels 48 to avoid installing electric lighting in the housing 36.

In order to ensure an efficient filtering of the air and recycling of the absorbent material, it is preferable that the air filtering apparatus 10 comprises an actuation mean for the filtering media 14 so that the nozzles 32 can extract the absorbent material on the entire surface of the filtering media 14 and not just on specific portions. Actuating the nozzles 32 is undesired as it would be more complicated because of the ducts 33 and the frame 30.

The filtering apparatus 10 comprises a motor device 52 to actuate the support structure 16, namely the cylindrical drum 18. The air filtering apparatus 10 may comprise a motor directly engaging the central saft 20 and inducing a rotary motion on the shaft 20 and thereby on the cylindrical drum 18 given the reinforcements 22 linking the central shaft 20 to the cylindrical drum 18. While simple to implement, there is a significant loss of torque as the driving force is applied on the central shaft 20 meaning at a distance from the main body of the cylindrical drum 18. According to an embodiment of the present disclosure, the filtering apparatus 10 comprises a motor device 52 directly engaging the cylindrical drum 18 and inducing a rotary motion directly on the cylindrical drum 18 and not at a distance.

For this, the filtering apparatus 10 comprises a driving mean and a driven mean engaging one another and arranged at the periphery of the cylindrical drum 18. As illustrated in FIG. 2, the cylindrical drum 18 comprises at one of its longitudinal ends a ring gear 50, or annular gear, with the teeth being extending radially inwards or radially outwards of the ring gear 50, here inwards. The air filtering apparatus 10 comprises a motor device 52 to actuate the ring gear 50 and induce a rotary motion to the cylindrical drum 18. The motor device 52 comprises a motor 54, such as an electric motor, driving in rotation, for example with a shaft, a geared plate 56, or pinion, which is engaging the teeth of the ring gear 50. The motor 54 actuates the plate 56 which in turns transmit this rotary motion to the ring gear 50 and cylindrical drum 18. The torque and power supplied by the motor 54 is directly transmitted to the main body of the cylindrical drum 18. The disclosure is not limited to this type of transmission. For instance the motor device can be selected from and not limited to a spur gear system, a worm drive, a belt transmission or an epicyclic gearing or planetary gearing. Similarly, the disclosure is not limited to these shapes, the gears can be selected from and not limited to spur gears, bevel gears, crown gears, spiral bevel gears, helical gears or herringbone gears. The present disclosure encompasses embodiments where elements such as gaskets are arranged between the cylindrical drum 18 and the ring gear 50. In other words, when saying that the motor device 52 is directly engaging the cylindrical drum 18 and inducing a rotary motion directly on the cylindrical drum 18, it encompasses embodiments where additional elements such as gaskets or additional gears are arranged between the motor device 52 and the cylindrical drum 18. With such an arrangement, the torque of the motor device 52 is directly transmitted to the cylindrical drum 18, more precisely to the main body and lateral face of the cylindrical drum 18, thereby reducing torque loss and the cylindrical drum 18 can be rotated with more power. In other words, by connecting the motor device 52 directly to the cylindrical drum 18, the needed torque to induce a rotation of said cylindrical drum 18 is minimized. Another way to say it, the torque is used or applied where it's really needed meaning on the outer surface of the cylindrical drum 18, i.e. a direct force flow. The geared plate 56 being stiff and stable, it makes the transfer of torque and inducing a rotation of said drum more precise.

Given that the cylindrical drum 18 can be rotated with more power, it is possible to approach the nozzle(s) 32 closer to the surface of the filtering media 14. Conventional nozzles are usually placed about 5,0 mm distant of the filtering media's 14 surface, but because of the better torque and driving force of the motor device 52 onto the cylindrical drum 18, it is possible to arrange the nozzles 32 between about 0,5 mm to about 4,0 mm remote from the filtering media's 18 surface, preferably between about 0,5 mm and about 3,0 mm, more preferably between about 0,5 and about 2,0 mm, even more preferably between about 0,5 mm and 1,0 mm, distant of the filtering media 14. The vacuum, or suction, induced by recycling pump 34 will not affect the rotation of the cylindrical drum 18 at these distances.

With the nozzles 32 being placed closer of the filtering media 14, the absorbent material is thereby extracted and recycled more efficiently. This ensures not only saving in raw material, but also increases the longevity of the filtering media 14 as it gets clogged less often.

As illustrated in FIG. 2, the nozzles 32 are not arranged at the same height, with respect to the cylindrical drum 18 in a functioning, or filtering, position. In other words, two or more nozzles 32 are not aligned with respect to the longitudinal axis of the cylindrical drum 18 *i.e.* with respect to the axis of the central shaft 20. The nozzles 32 and the vacuum induced by the recycling pump 34 is thereby spread out, meaning not at one single rotational position, or not at one vertical position, which has a lesser impact on the rotational movement of the cylindrical drum 18. The nozzles 32 can also be brought closer to the filtering media 14, between about 0,5 mm to 4,0 mm, preferably between 0,5 mm and 1,0 mm thanks to this arrangement of nozzles 32 having a lesser impact on the rotation of the drum 18. Furthermore, absorbent material arrives to the air filtering apparatus in different amounts at different times, for example if there's a malfunction in the process line. Having the nozzles arranged at different height enables to better handle the different arrivals of absorbent material, as it will not reach the same longitudinal portions of the cylindrical drum 18.

As illustrated in FIG. 2, it is preferable that the nozzles are arranged on a same side or half of the cylindrical drum, this is mainly to simplify the ducts 33 and pump 34 arrangement.

As illustrated in FIG. 2, the motor device 52 is elevated by a platform 58 for example here a plate that is bolted to the frame 30 or its reinforcement struts. The cylindrical drum 18, being actuated in rotation, is preferably elevated and maintained above the ground, by the frame 30 structure, hence the motor device 52 actuating said cylindrical drum 18 is also preferably elevated.

The cylindrical drum 18 comprises a first 49 and second 60 longitudinal ends. At the first longitudinal end 49, the cylindrical drum 18 comprises a mean that can be driven or actuated, in this case a ring gear 50 that it can be actuated in rotation by the motor device 52. In other words, the first longitudinal end 49 comprises a mean enabling the cylindrical drum 18 to be rotated. At the opposite longitudinal end, meaning the second longitudinal end 60, the cylindrical drum 18 is preferably closed-off, meaning that an airflow cannot pass through the second longitudinal end 60. The second longitudinal end 60 comprises an air-impermeable mean such as a metal plate preventing the air from passing through. The air entering through the inlets 12 is deflected by the plate and is forced to flow through the filtering media 14 and mesh grid 19. In this embodiment, the filtering media 14 is in the shape or form of a rectangular mat of filtering material that is arranged around the cylindrical drum 18 and is secured or fastened to said drum 18 by the zipper 24 and/or straps 26. In other words, the cylindrical drum 18 is substantially hollow, not taking into account the reinforcements 22 and central shaft 20, and comprises two longitudinal ends defining the bases of the cylinder, one of which is sealed, and a mesh or grid 19 defining the lateral face of the cylinder.

The disclosure is not limited to this embodiment, for instance, the second longitudinal end 60 can also comprise a mesh or grid and the filtering media 14 is in the shape or form of a sleeve of filtering material that is slid onto the cylindrical drum 18.

The one or more inlets 12 are preferably arranged in the bottom, with respect to height, of the housing 36. The air coming from the process line contains absorbent material and that absorbent material can accumulate on the floor on the housing 36. By arranging the inlet(s) 12 in the bottom, the incoming air can pick up some absorbent material that has accumulated on the floor and convey it to the filtering media 14 to be reinjected in the process line *via* the recycling pump 34.

The filtering media 14 is a media filter using a bed of fibrous or porous material such as textile fabric or foam and removes the solid particulate such as the absorbent material from the air. The filtering media 14 can for example be composed of paper, foam, woven or nonwoven fabric or cotton.

The disclosure is not limited to the embodiment illustrated in FIG. 1. The process line 62 may comprise one, two, three or all the assembly modules 64 fluidically connected to the basic pump 71. The process line 62 may comprise an assembly module 64 that is directly connected to the filtering apparatus 10 and main pump 38, meaning with no booster pump or basic pump 71 in-between. The process line 62 may comprise two, three, four, five or more channels with one or more interconnections 76. All the assembly module 64 may be connected to the actuator 47 and configured to send it a feedback signal 51, or electronic signal 51.

The assembly modules 64 are elements, components, modules, units or stations of the process line 62 where different components of the absorbent article are assembled, transferred, glued, *etc..* In other terms, an assembly module 64 is a part, or portion, of the process line 62 where the absorbent article undergoes a transformation be it a spatial transformation e.g. a transfer, rotation or folding, *etc*.; or be it a structural transformation *e.g.* cutting, adding glue, a web or leg cuffs, *etc..* For example the assembly modules 64 comprise a pad-cut unit anvil roller with a corresponding knife roller; the drum former for the absorbent core, an insert repitch unit or a transfer drum pad to rotary unit; a pad rotary unit; a final-cut unit anvil with corresponding knife roller or a final rotary unit which require vacuum and are preferably associated with a booster pump. The assembly modules 64 can also comprise for example glue or adhesive dispensers, rollers for applying elastics, frontal tapes or back ears, leg cuffs applicators, conveyor belts, transfer rollers, folding stations, *etc.* where vacuum is not necessary and are not associated with a booster pump 72. The process line 62 can also comprise assembly modules 64 that are not connected to the air circulation system and/or not connected to the air filtering apparatus 10.

An assembly module 64 can also be associated with more than one booster pump 72. For example, as illustrated in FIG. 4, an assembly module 64 can be in fluidically connection with one or more booster pump 72. These booster pumps 72 can be arranged in series or in parallel. For instance the assembly module 64f is connected with two boosters pump 72s arranged in series whereas the assembly module 64g is connected with three boosters pump 72p arranged in parallel with an optional three-way valve 73 to orientate the airflow properly, shutting-off one or more pumps 72p being an alternative mean to orientate the airflow. These arrangements enable to have more flexibility and control in the adjustment of the vacuum generated by the booster pumps. The booster pump 72 preferably have different flow rate. For instance, the process line 62 can comprise an assembly module 64 connected to two booster pumps 72 in parallel each having a volume flow rate of 280 m³/h, another assembly module 64 connected to one booster pump 72 having a volume flow rate of 330 m³/h and another assembly module 64 connected to three booster pumps 72 in series having a volume flow rate of 530, 318 and 250 m³/h respectively. Such arrangement gives more flexibility to the manufacturing of absorbent article and the main pump and basic pump can work at lower power consumptions, *i.e.* lower kWh.

The process line 62 can also comprise communication means, such as electronic signals, between each booster pumps 72 and the main pump 38 and/or the basic pump 71. For example, if one booster pump 72 is malfunctioning, it can send a signal to the basic pump 71, the latter increasing slightly it's power and ensuring the same level of vacuum in said assembly module 64.

The number of assembly modules 64 can be greater than the number of booster pumps 72 as illustrated in FIG. 1 or the number of booster pump 72 can be greater than the number of assembly modules 64 as illustrated in FIG. 4.

## Claims

1. Process line (62) for manufacturing absorbent articles comprising a plurality of assembly modules (64), the process line (62) comprising at least one channel (70) fluidically connecting at least one assembly module (64) and a main pump (38), said main pump being arranged downstream, with respect to the airflow, of said assembly module (64), wherein at least one booster pump (72) is arranged between an assembly module (64) and said main pump (38), said at least one channel (70) fluidically connecting said assembly module (64), booster pump (72) and main pump (38), wherein the volume flow rate of the main pump (38) is at least twenty times greater than the volume flow rate of any of the booster pumps (72).

2. Process line (62) for manufacturing absorbent articles according to claim 1, wherein the number of assembly modules (64) is greater than the number of booster pumps (72).

3. Process line (62) for manufacturing absorbent articles according to any of the preceding claims, wherein the process line (62) comprises a filtering apparatus (10) arranged upstream of main pump (38), with respect to the airflow.

4. Process line (62) for manufacturing absorbent articles according to any of the preceding claims, wherein the volume flow rate of the main pump (38) is comprised between 20 000 m³/h and 50 000 m³/h and the volume flow rate of any booster pump (72) is comprised between 100 m³/h and 1 000 m³/h.

5. Process line (62) for manufacturing absorbent articles according to any of the preceding claims, wherein the process line (62) comprises at least two channels (70).

6. Process line (62) for manufacturing absorbent articles according to claim 6, wherein said at least two channels (70) comprise an interconnection (76) where two channels fuse into a single channel, the interconnection (76) being arranged upstream of the filtering apparatus (10).

7. Process line (62) for manufacturing absorbent articles according to any of the preceding claims, wherein the process line (62) comprises a basic pump (71) arranged upstream of the main pump (38) and downstream of the booster pump (72), said at least one channel (70) fluidically connecting said assembly module (64), booster pump (72), basic pump (71) and main pump (38).

8. Process line (62) for manufacturing absorbent articles according to claim 7, wherein the volume flow rate of the main pump (38) is between 0.8 and 2 times greater than the volume flow rate of the basic pump (71), the volume flow rate of the basic pump (71) being comprised between 20 000 m³/h and 40 000 m³/h.

9. Process line (62) for manufacturing absorbent articles according to any of the preceding claims, wherein the process line (62) comprises communication means between the booster pumps (72) and the main pump (38) and/or basic pump (71) wherein a booster pump (72) is configured to send electronic signals to the main pump (38) and/or basic pump (71).

10. Process line (62) for manufacturing absorbent articles according to claim 3 or any of the claims 4 to 9 dependent of claim 3, wherein the filtering apparatus (10) comprises:
a. a housing (36) comprising at least one inlet (12);
b. a filtering media (14) arranged downstream with respect to the airflow of the at least one inlet (12);
c. a support structure (16) comprising a cylindrical drum (18) comprising a first and second longitudinal end (49,60), the filtering media (14) being secured to said cylindrical drum (18);
d. a motor device (52) to rotate the cylindrical drum (18); and
e. a first pump (38);
wherein the motor device (52) comprises a driving mean (56) and the cylindrical drum (18) comprises the corresponding driven mean (50) arranged at the periphery of one of the longitudinal ends (49,60) of the cylindrical drum (18), the driving mean (56) actuating the driven mean and cylindrical drum (18).

11. Process line (62) for manufacturing absorbent articles according to claim 10, wherein the driving mean (56) comprises a geared plate (56) and the driven mean (50) comprises a ring gear (50), the teeth of the geared plate (56) engaging the teeth of the ring gear (50).

12. Process line (62) for manufacturing absorbent articles according to any of the claims 3, 10 or 11, wherein the filtering apparatus (10) comprises a recycling pump (34) arranged to suck up a lesser portion of the air incoming in the housing (36) than the main pump (38).

13. Process line (62) for manufacturing absorbent articles according to claim 12, wherein the filtering apparatus (10) comprises a least one nozzle (32) arranged in proximity of the filtering media (14) and at least one duct (33) linking said nozzle (32) to the second pump (34), wherein two or more nozzles (32) are not aligned with respect to the longitudinal axis of the cylindrical drum (18).

## Patentansprüche

1. Prozesslinie (62) zum Herstellen absorbierender Artikel, umfassend eine Vielzahl von Zusammenbaumodulen (64), die Prozesslinie (62) umfassend mindestens einen Kanal (70), der mindestens ein Zusammenbaumodul (64) und eine Hauptpumpe (38) fluidisch verbindet, wobei die Hauptpumpe hinsichtlich des Luftstroms nachgeschaltet des Zusammenbaumoduls (64) angeordnet ist, wobei zwischen einem Zusammenbaumodul (64) und der Hauptpumpe (38) mindestens eine Zusatzpumpe (72) angeordnet ist, wobei der mindestens eine Kanal (70) das Zusammenbaumodul (64), die Zusatzpumpe (72) und die Hauptpumpe (38) fluidisch verbindet, wobei der Volumendurchfluss der Hauptpumpe (38) mindestens zwanzigmal größer als der Volumendurchfluss einer beliebigen der Zusatzpumpen (72) ist.

2. Prozesslinie (62) zum Herstellen absorbierender Artikel nach Anspruch 1, wobei die Anzahl von Zusammenbaumodulen (64) größer als die Anzahl von Zusatzpumpen (72) ist.

3. Prozesslinie (62) zum Herstellen absorbierender Artikel nach einem der vorstehenden Ansprüche, wobei die Prozesslinie (62) eine Filtereinrichtung (10), die hinsichtlich des Luftstroms vorgeschaltet der Hauptpumpe (38) angeordnet ist, umfasst.

4. Prozesslinie (62) zum Herstellen absorbierender Artikel nach einem der vorstehenden Ansprüche, wobei der Volumendurchfluss der Hauptpumpe (38) zwischen 20 000 m³/Std. und 50 000 m³/Std. beträgt und der Volumendurchfluss einer beliebigen Zusatzpumpe (72) zwischen 100 m³/Std. und 1 000 m³/Std. beträgt.

5. Prozesslinie (62) zum Herstellen absorbierender Artikel nach einem der vorstehenden Ansprüche, wobei die Prozesslinie (62) mindestens zwei Kanäle (70) umfasst.

6. Prozesslinie (62) zum Herstellen absorbierender Artikel nach Anspruch 6, wobei die mindestens zwei Kanäle (70) eine Verbindung (76), an der zwei Kanäle zu einem einzigen Kanal verschmelzen, umfassen, wobei die Verbindung (76) vorgeschaltet der Filtereinrichtung (10) angeordnet ist.

7. Prozesslinie (62) zum Herstellen absorbierender Artikel nach einem der vorstehenden Ansprüche, wobei die Prozesslinie (62) eine Basispumpe (71), die vorgeschaltet der Hauptpumpe (38) und nachgeschaltet der Zusatzpumpe (72) angeordnet ist, wobei der mindestens eine Kanal (70) das Zusammenbaumodul (64), die Zusatzpumpe (72), die Basispumpe (71) und die Hauptpumpe (38) fluidisch verbindet.

8. Prozesslinie (62) zum Herstellen absorbierender Artikeln nach Anspruch 7, wobei der Volumendurchfluss der Hauptpumpe (38) zwischen 0,8- und 2-mal größer als der Volumendurchfluss der Basispumpe (71) ist, wobei der Volumendurchfluss der Basispumpe (71) zwischen 20 000 m³/Std. und 40 000 m³/Std. beträgt.

9. Prozesslinie (62) zum Herstellen absorbierender Artikel nach einem der vorstehenden Ansprüche, wobei die Prozesslinie (62) Kommunikationsmittel zwischen den Zusatzpumpen (72) und der Hauptpumpe (38) und/oder der Basispumpe (71) umfasst, wobei eine Zusatzpumpe (72) konfiguriert ist, um elektronische Signale an die Hauptpumpe (38) und/oder die Basispumpe (71) zu senden.

10. Prozesslinie (62) zum Herstellen absorbierender Artikel nach Anspruch 3 oder einem der Ansprüche 4 bis 9, abhängig von Anspruch 3, wobei die Filtereinrichtung (10) umfasst:
a. ein Gehäuse (36) umfassend mindestens einen Einlass (12);
b. ein Filtermedium (14), das hinsichtlich des Luftstroms nachgeschaltet des mindestens einen Einlasses (12) angeordnet ist;
c. eine Trägerstruktur (16), umfassend eine zylindrische Trommel (18), umfassend ein erstes und ein zweites Längsende (49,60), wobei das Filtermedium (14) an der zylindrischen Trommel (18) gesichert ist;
d. eine Motorvorrichtung (52), um die zylindrische Trommel (18) zu drehen; und
e. eine erste Pumpe (38);
wobei die Motorvorrichtung (52) ein Antriebsmittel (56) umfasst und die zylindrische Trommel (18) das entsprechende angetriebene Mittel (50) umfasst, das an dem Umfang eines der Längsenden (49,60) der zylindrischen Trommel (18) angeordnet ist, wobei das Antriebsmittel (56) das angetriebene Mittel und die zylindrische Trommel (18) in Bewegung versetzt.

11. Prozesslinie (62) zum Herstellen absorbierender Artikel nach Anspruch 10, wobei das Antriebsmittel (56) eine verzahnte Platte (56) und das angetriebene Mittel (50) ein Hohlrad (50) umfasst, wobei die Zähne der verzahnten Platte (56) in die Zähne des Hohlrads (50) eingreifen.

12. Prozesslinie (62) zum Herstellen absorbierender Artikel nach einem der Ansprüche 3, 10 oder 11, wobei die Filtereinrichtung (10) eine Recyclingpumpe (34), die angeordnet ist, um einen geringeren Anteil der in das Gehäuse (36) eintretenden Luft als die Hauptpumpe (38) anzusaugen, umfasst.

13. Prozesslinie (62) zum Herstellen absorbierender Artikel nach Anspruch 12, wobei die Filtereinrichtung (10) mindestens eine Düse (32), die nahe dem Filtermedium (14) angeordnet ist, und mindestens eine Leitung (33), die die Düse (32) mit der zweiten Pumpe (34) verknüpft, umfasst, wobei zwei oder mehr Düsen (32) hinsichtlich der Längsachse der zylindrischen Trommel (18) nicht ausgerichtet sind.

## Revendications

1. Ligne de traitement (62) pour la fabrication d'articles absorbants comprenant une pluralité de modules d'assemblage (64), la ligne de traitement (62) comprenant au moins un canal (70) reliant fluidiquement au moins un module d'assemblage (64) et une pompe principale (38), ladite pompe principale étant agencée en aval, par rapport au flux d'air, dudit module d'assemblage (64), dans laquelle au moins une pompe de surpression (72) est agencée entre un module d'assemblage (64) et ladite pompe principale (38), ledit au moins un canal (70) reliant fluidiquement ledit module d'assemblage (64), la pompe de surpression (72) et la pompe principale (38), dans laquelle le débit volumétrique de la pompe principale (38) est au moins vingt fois supérieur au débit volumétrique de l'une quelconque des pompes de surpression (72).

2. Ligne de traitement (62) pour la fabrication d'articles absorbants selon la revendication 1, dans laquelle le nombre de modules d'assemblage (64) est supérieur au nombre de pompes de surpression (72).

3. Ligne de traitement (62) pour la fabrication d'articles absorbants selon l'une quelconque des revendications précédentes, dans laquelle la ligne de traitement (62) comprend un appareil de filtrage (10) agencé en amont de la pompe principale (38), par rapport au flux d'air.

4. Ligne de traitement (62) pour la fabrication d'articles absorbants selon l'une quelconque des revendications précédentes, dans laquelle le débit volumétrique de la pompe principale (38) est compris entre 20 000 m³/h et 50 000 m³/h et le débit volumétrique de l'une quelconque des pompes de surpression (72) est compris entre 100 m³/h et 1 000 m³/h.

5. Ligne de traitement (62) pour la fabrication d'articles absorbants selon l'une quelconque des revendications précédentes, dans laquelle la ligne de traitement (62) comprend au moins deux canaux (70).

6. Ligne de traitement (62) pour la fabrication d'articles absorbants selon la revendication 6, dans laquelle lesdits au moins deux canaux (70) comprennent une interconnexion (76) où deux canaux fusionnent en un seul canal, l'interconnexion (76) étant agencée en amont de l'appareil de filtrage (10).

7. Ligne de traitement (62) pour la fabrication d'articles absorbants selon l'une quelconque des revendications précédentes, dans laquelle la ligne de traitement (62) comprend une pompe de base (71) agencée en amont de la pompe principale (38) et en aval de la pompe de surpression (72), ledit au moins un canal (70) reliant fluidiquement ledit module d'assemblage (64), la pompe de surpression (72), la pompe de base (71) et la pompe principale (38).

8. Ligne de traitement (62) pour la fabrication d'articles absorbants selon la revendication 7, dans laquelle le débit volumétrique de la pompe principale (38) est entre 0,8 et 2 fois supérieur au débit volumétrique de la pompe de base (71), le débit volumétrique de la pompe de base (71) étant compris entre 20 000 m³/h et 40 000 m³/h.

9. Ligne de traitement (62) pour la fabrication d'articles absorbants selon l'une quelconque des revendications précédentes, dans laquelle la ligne de traitement (62) comprend des moyens de communication entre les pompes de surpression (72) et la pompe principale (38) et/ou la pompe de base (71), dans laquelle une pompe de surpression (72) est configurée pour envoyer des signaux électroniques à la pompe principale (38) et/ou à la pompe de base (71).

10. Ligne de traitement (62) pour la fabrication d'articles absorbants selon la revendication 3 ou l'une quelconque des revendications 4 à 9 dépendant de la revendication 3, dans laquelle l'appareil de filtrage (10) comprend :
a. un boîtier (36) comprenant au moins une entrée (12) ;
b. un média filtrant (14) agencé en aval par rapport au flux d'air de l'au moins une entrée (12) ;
c. une structure de support (16) comprenant un tambour cylindrique (18) comprenant une première et une seconde extrémité longitudinale (49,60), le média filtrant (14) étant fixé audit tambour cylindrique (18) ;
d. un dispositif moteur (52) pour faire tourner le tambour cylindrique (18) ; et
e. une première pompe (38) ;
dans laquelle le dispositif moteur (52) comprend un moyen d'entraînement (56) et le tambour cylindrique (18) comprend le moyen entraîné correspondant (50) agencé à la périphérie de l'une des extrémités longitudinales (49,60) du tambour cylindrique (18), le moyen d'entraînement (56) actionnant le moyen entraîné et le tambour cylindrique (18).

11. Ligne de traitement (62) pour la fabrication d'articles absorbants selon la revendication 10, dans laquelle le moyen d'entraînement (56) comprend une plaque dentée (56) et le moyen entraîné (50) comprend une couronne dentée (50), les dents de la plaque dentée (56) venant en prise avec les dents de la couronne dentée (50).

12. Ligne de traitement (62) pour la fabrication d'articles absorbants selon l'une quelconque des revendications 3, 10 ou 11, dans laquelle l'appareil de filtrage (10) comprend une pompe de recyclage (34) agencée pour aspirer une partie moindre de l'air entrant dans le boîtier (36) que la pompe principale (38).

13. Ligne de traitement (62) pour la fabrication d'articles absorbants selon la revendication 12, dans laquelle l'appareil de filtrage (10) comprend au moins une buse (32) agencée à proximité du média filtrant (14) et au moins un conduit (33) reliant ladite buse (32) à la seconde pompe (34), dans laquelle deux ou plusieurs buses (32) ne sont pas alignées par rapport à l'axe longitudinal du tambour cylindrique (18).
